Europäisches Patentamt

European Patent Office

Office européen des brevets

(11) Publication number: **0 487 187 A1**

## EUROPEAN PATENT APPLICATION

(21) Application number: 91307340.9

(51) Int. Cl.5: **A23L 1/09**, A23L 1/308

(22) Date of filing: 09.08.91

(30) Priority: **15.11.90 JP 309044/90**

(43) Date of publication of application:
**27.05.92 Bulletin 92/22**

(84) Designated Contracting States:
**AT BE CH DE DK ES FR GB GR IT LI LU NL SE**

(71) Applicant: MATSUTANI CHEMICAL
INDUSTRIES CO. LTD.
3-Banchi, 5-chome, Kita Itami
Itami-shi, Hyogo-ken(JP)

(72) Inventor: Ohkuma, Kazuhiro
4-7, Yayoigaoka 3-chome
Sanda-shi, Hyogo-ken(JP)
Inventor: Matsuda, Isao
717-1, Noma Aza Raifukuzi
Itami-shi, Hyogo-ken(JP)
Inventor: Hanno, Yoshio
52-401, Ogino 3-chome
Itami-shi, Hyogo-ken(JP)

(74) Representative: Low, Peter John et al
WILSON, GUNN & ELLIS 41-51 Royal
Exchange Cross Street
Manchester, M2 7DB(GB)

(54) Low caloric foods and drinks.

(57) A low caloric food or drink comprising a caloric maltodextrin obtained by dissolving a pyrodextrin prepared through heating a starch mixed with mineral acid into water, and reacting alpha-amylase on the pyrodextrin.

## BACKGROUND OF THE INVENTION

1. Field of the Invention:

The present invention relates to foods and drinks containing, as a component, maltodextrin obtained by treating pyrodextrin with enzyme, and to a method for producing such foods and drinks.

2. Description of Prior Art:

Recent years, living standard has been remarkably improved in Japan and, in particular, eating habits have come to substantially the same level to those in Western countries. As a result, average life of Japanese has been prolonged, thereby bringing about rapidly an aging society which, in turn, results in structural change of diseases sharply increasing diseases of adult people. Thus, promotion of health is now one of the matters of greatest concern. In this connection, the Health and Welfare Ministry of Japan published a proposal of "Guideline of Eating Habits for Promotion of Health" in 1985 with regard to eating habits of Japanese people in general, and in which it was pointed out that one of the problems pertaining to Japanese eating habits was "excessive intake of energy."

On the other hand, starch itself and most of modified starches such as pregelatinized starch pyrodextrin, starch derivative, glucose, corn sirup solid and maltodextrin are actually employed in large quantity. Caloric value of these starch products, however, mounts to about 400Kcal/100g, and accordingly only pyrodexitrin is known among the starch products as a useful low caloric food material capable of saving the mentioned "excessive intake of energy."

The inventors of the present application have been aggressively engaged in research and development of dietary fibers, and based on the results thereof already filed a patent application titled "method for preparing dextrin containing high percentage of dietary fibers" and others. Further, as a result of studies on physiological functions of this dextrin, the inventors recognized that the dextrin performs such useful effects as large bowel regulation, improvement in hyper cholesteremia, saving insulin secretion, reduction of blood pressure, and based on which application of such dextrin was already filed in the form of food composite. Having recognized furthermore that calorie of this dextrin containing dietary fibers is low, the present invention has been made.

## SUMMARY OF THE INVENTION

Accordingly, an object of the present invention is to provide a low caloric food utilizing dextrin containing dietary fibers.

The foregoing object is accomplished by providing a food containing maltodextrin of which requirements to be a low caloric food material are defined.

The maltodextrin employed as a component of low caloric food in accordance with the invention is produced in the following methods:

(1) A method comprising the steps of: heating a starch, preferably a potato starch, by treatment with mineral acid thereby obtaining a pyrodextrin; reacting alpha-amylase with the pyrodextrin obtained; and refining the pyrodextrin thus treated with alpha-amylase by filtering according to normal method.

(2) A method comprising the steps of: reacting alpha-amylase with an ordinary pyrodextrin; applying glucoamylase to the hydrolyzate; refining the resulted hydrolyzate by filtering; and extracting high caloric section.

Described first is the above method (1).

It is preferred to use a quite limited kind of pyrodextrin as a raw material. That is, a potato starch is a suitable starch material. It is essential to add a mineral acid as a catalyst in the heating step. Various mineral acids are known, and from the viewpoint of use in food, it is particularly desirable to adopt hydrochloric acid. In the pyrodextrin thus obtained, caloric value should be not more than 240Kcal/100g in view of application to prepare a low caloric food. In other words, pyrodextrin whose content corresponds to not more than 60% of 400Kcal/100g of ordinary starch or starch product should be used as the raw material. In this respect, it is to be noted that caloric value of white dextrin popularly used in food and medical field exceeds far from the mentioned 240Kcal/100g, and is not suitable for the purpose of achieving low calorie. On the other hand, if caloric value is less than 160Kcal/100g, the food even though hydrolized with alpha-amylase exhibits somewhat stimulative taste. Moreover, load in the refining step after hydrolysis with alpha-amylase is enhanced thereby making it impossible to carry out mass production. Thus, such dextrin of excessively low calorie value is not suitable, either.

Various alpha-amylases can be widely employed in the present invention including bacterial alpha-amylase. From the viewpoint of reducing calorie of dextrin in the object, it is particularly preferable to use "Termamyl" (produced by Novo Ind.) and "Klaistase T-5"(produced by Daiwa Kasei Co.).

The low caloric dextrin thus obtained has usually a caloric value within a range of not more than 240Kal/100g and not less than 160Kcal/100g which is quite preferable as a low caloric food material.

Potato starch treated with mineral acid and heated according to normal method is preferably employed as a pyrodextrin treated with mineral acid to be used in the mentioned method (1). As for the amount of mineral acid, about 1 % aqueous solution is added to the starch by several % (3 to 5%). To satisfy the heating requirement, considering that an acidified aqueous solution was already added, the starch and acid are evenly mixed by stirring and maturing in a mixer, then the mixture is preliminarily dried at 100 to 120°C to reduce moisture content to about 5%, and heated at a temperature of 150 to 220°C for 1 to 5 hours. Treatment of the pyrodextrin with alpha-amylase can be carried out according to normal method, and in which about 30 to 45% aqueous solution of pyrodextrin is prepared with its pH adjusted to 4.5 to 6.5, then alpha-amylase is added to the pyrodextrin by 0.05 to 0.2%, and the pyrodextrin is held for 30 min to 2 hours at 85 to 100°C, i.e., a reaction temperature of alpha-amylase (this temperature depends on the type of alpha-amylase. In the next step, reaction of alpha-amylase is suspended by increasing the temperature to about 120°C (i.e., deactivation temperature of alpha-amylase). In this step, it is also possible that pH is reduced to a value of deactivating alpha-amylase, i.e., pH 4 or so. After completing the reaction of alpha-amylase, activated charcoal is added for the purpose of removing insoluble matter, color, etc. Then filtration is carried out by means of known filter press, precoat filter, or the like. Thereafter , salts and coloring matter in the solution are removed by ion exchanger resins. Usually, a cation exchanger resin, an anion exchanger resin and a resin mixing both types are applied in this order.

Described now is the mentioned method (2).

In this method, not only pyrodextrin prepared from potato starch but also ordinary pyrodextrins can be widely employed. Any ordinary pyrodextrin prepared from various starches can be employed. Treatment with alpha-amylase is carried out in the same manner as the foregoing method (1), but in this method (2), after completing such treatment, a further treatment with glucoamylase is required, and in which normal conditions for such treatment with gluycoamylase is adopted. For example, a solution temperature is reduced to about 55°C with its pH adjusted to about 5.5, then 0.05 to 0.2 % by weight of ordinary glucoamylase is added to the original pyrodextrin, and reaction takes place for 24 to 48 hours keeping the solution temperature. This reaction is to decompose small molecules such as oligosaccharide existing in the solution to glucose. Then the reaction of glucoamylase is completed at 80°C, for example.

Subsequently, filtration and refining are once carried out according to normal method. Conventional filtration and refining with the use of ion exchanger resin is satisfiable.

High calorie fraction is then separated and removed using ion exchanger resin by chromatographic method and/or organic solvent method. For that purpose, any conventional strongly acidic cation exchanger resin sold on the market can be widely used.

Preferable as concrete examples are Amberlite IR-116, IR-118, IR-120B, XT-1022E, XT-471F (all manufactured by Organo), Diaion SK-1B, SK-102, SK-104, SK-106, SK-110, SK-112, SK-116, FR-01 (all manfactured by Mitsubishi Chemicals), and XFS-43281,00, XFS-43280.00, XFS-43279.00, XFS-43278.00 (all manufactured by Dow Chemicals).

These resins are preferably dealt with as alkaline metale type or alkaline earth metal type before their uses. It is preferable to adjust the rate of flow at the time of a colum fluid according to a resin to be used. The rate of flow of the fluid is preferably in the range of SV = 0.1 to 0.6. The rate of flow out of the above range tends to deteriorate the workability and separation. The temperature at the time of running of the fluid is preferably in the range from 20°C to 70°C, and a temperature below this range will deteriorate the separation and make the viscosity of fluid get high, thereby giving a negative influence on the fluid, while a temperature exceeding this range will cause the fluid to be tanned and deterirorate other quality characteristics.

The mentioned organaic solvent method is a separation method using solvent capable of dissolving digestible or low molecular weight components, and accordingly it is preferable to adopt any solvent capable of dissolving low molecular weight components. Preferable as a representative example of such solvent is ethanol.

In the mentioned method (2), since reaction with glucoamylase takes place after hydrolysis with alpha-amylase, not only the pyrodextrin prepared from potato starch is preferable but also various other pyrodextrins can be equivalently used to obtain a dextrin of desired low calorie value, i.e., not more than 240Kcal/100g, more preferably, not more than 160Kca1/100g. Since glucoamylase is additionally applied after hydrolysis with alpha-amylase in the method (2), stimulant matters in the object dextrin of not more than 160Kcal/100g can be also successfully separated by normal method in the steps of filtration and refining.

Several examples are hereinafter described in order to show clearly the features of the present invention.

### Example 1

50ml of 1% hydrochloric acid solution was applied by spraying on each 1Kg of various starches sold on the market and mixed evenly by means of a mixer, placed on an aluminum vat, dried preliminarily for 1 hour in a drier, then heated at a temperature of 150°C for 2 hours. Hot water in twice as much as each pyrodextrin was added to each pyrodextrin which was then neutralized to pH5.8 with 1N sodium hydroxide, then 0.1% "Termamyl" was added to each solution to react at a temperature of 95°C for 1 hour, and further heated up to 115°C to complete the reaction. Subsequently, each solution was filtered and decolorized and concentrated in vacuo a concentration of 30%. Thereafter, caloric value of each solution together with intermediate products was determined, and transparency of each sample solution was measured. Table 1 shows the result.

TABLE 1

| Material starches | Potato starch | Tapioca | Corn starch |
|---|---|---|---|
| Kcal/100g of pyrodextrin | 168 | 204 | 194 |
| Kcal/100g after decomposition with alpha-amylase | 180 | 212 | 200 |
| Transparency of solution after refining | transparent | transparent but reddish | opaque |

### Example 2

50ml of 1% hydrochloric acid solution was applied by spraying on each 1Kg of potato starches sold on the market and mixed evenly by means of a mixer, placed on an aluminum vat, dried preliminarily for 1 hour in a drier, then heated at a temperature of 150°C for 5 hours while picking up 800g of sample every hours. Hot water in twice as much as each pyrodextrin was added to each pyrodextrin which was then neutralized to pH5.8 with 1N sodium hydroxide, then 0.1% "Termamyl" was added to each solution to react at a temperature of 95°C for 1 hour, and futher heated up to 115°C to complete the reaction.
Subsequently, the solution decolorized and filtered by normal method was subject to a deionization test using mixed bed ion exchanger resins. For acknowledging deionization amount of solution, a point of time when chloride ion began to leak into the effluent, was considered end point. Caloric value of the deionized solution was determined after being concentrated in vacuo to 30%, and flavor of each solution was measured by sensory test. Table 2 shows the result.

TABLE 2

| Heating time | 1 | 2 | 3 | 4 | 5 |
|---|---|---|---|---|---|
| Amount of deionized effluent by ion exchanger resin (ml/ml resin) | 7.31 | 5.54 | 4.30 | 3.71 | 2.69 |
| Kcal/100g | 305 | 248 | 176 | 164 | 156 |
| Flavor (stimulant taste) | good | good | good | good | excessively stimulant |

### Example 3

0.1% "Klaistase KD" was added to each 400g of pyrodextrin obtained in the foregoing Example 2 and reacted at 85°C. Other treatments and conditions were the same as those described above. Table 3 shows the result of determination of caloric values.

TABLE 3

| Heating time | Kcal/100g |
|---|---|
| 1 | 300 |
| 2 | 268 |
| 3 | 200 |
| 4 | 192 |
| 5 | 190 |

It is understood from Table 1 that caloric value of 3 kinds of starches are more or less 200 Kcal/100g; that a transparent solution can be obtained when using potate starch; that use of tapioca starch results in a reddish solution; and that solution using corn starch is opaque. This means that the latter two starches are not suitable for food.

It is also understood from Table 2 that, though caloric value reduces preferably in proportion to the length of heating time, but that the capacity of ion exchanger resin reduces where calorie value comes down to less than 160Kcal/100g. Since the penetration is one of the important steps in refining process, such reduction of capacity is not desirable for mass production, and moreover there arises a futher disadvantage of stimulant taste impossible to remove even by ion exchanger resin, showing that is unpractical

Comparing the caloric values between Tables 2 and 3, it is understood that lower calorie is achieved in the dextrin prepared by adding "Termamyl", and therefore it is obviously preferable to use "Termamyl", as far as caloric value is in the mentioned range of 160 to 240Kcal/100g. In addition, measurement of the caloric value was carried out in the same manner as a later described embodiment.

Note that the low caloric dextrin achieved in accordance with the invention can be desirably applied to varieties of foods including, but not limited, breads and confectionaries such as cookie, doughnut, cake, bread; creams such as custard cream, cream, butter cream; and varieties of other foods such as chocolate, chewing gum, pudding, Bavarian, jelly, yoghurt, ice cream, juce, milk shake.

## DESCRIPTION OF THE PREFERRED EMBODIMENTS

Several embodiments in accordance with the present invention is hereinafter described. In these embodiments, measurement of caloric values were carried out in the following method by quantitative analysis.

(Method for measuring calorie)

Measurements were carried out by the following expression for "Measurement of Sugar Amount Having Physiological Combustion Value" issued on December 26, 1973, Medical Bulletin (JP) No.781.

Glucide having physiological combustion value:

Amount of sugar having physiological combustion value is calculated as follows: Sugar having physiological combustion value
= Total water-soluble reducting sugar
+ Enzymatic digestible sugar
+ Insoluble starch
+ Sorbitol
+ Xylose

(1) Reagent

Somogyi Copper Reagent:

71g of $Na_2HPO_4 \cdot 12H_2O$ and 40g of potassium sodium tartrate was dissolved into water, 100ml of 1NNaOH was added, then 80ml of 10%(W/V) $CuSO_4 \cdot 5H_2O$ and 410g of $Na_2SO_4 10H_2O$ were added to be dissolved, 10ml of INKIO solution was added, and 1000ml of solution was abtained by further adding water.

5

(2) Measurement of Total Water-soluble Reducing Sugar Preparation of solution to be tested:

If a sample includes oligosaccharides alone they are extracted using water or 80% ethanol, and if including polysaccharides, then oligosaccharides should be completely extracted using 80% ethanol. If extraction has been carried out using ethanol, then solvent should be eliminated in vacuo at 60°C, and residues be dissolved and diluted with water. In a sample not containing sucrose an aqueous solution of 5 to 15mg% glucose is used as a solution to be tested. On the other hand, in a sample containing sucrose, 0.1NHCl is added to the extract in the ratio of 3 to 10, then hydrolyzed in a boiling water bath for 30 min, cooled and neutralized, and an aqueous solution of 5 to 15mg% glucose is employed as a solution to be tested.

Procedure:

Adding 5ml of Somogyi cooper reagent to 5ml of solution to be tested, determination was carried out on the glucose by Somogi method.

(3) Preparation of Enzyme Solution

100ml of 0.2M acetic acid fubber solution (pH4.8) was added to 0.02 to 0.1g of commercial glucoamylase. After extraction by sufficiently shaking for 10 min, the solution was centrifuged and activity of supernatant was measured by the following method to dilute to 30/ml (produce $3\mu$ mole glucose per minute), futhermore a digestion test of soluble starch was carried out, thus an enzyme solution being obtained.

Measurement of enzyme activity:

The supernatant obtained as mentioned above was diluted to 1.0 to 50.0 mg% (equivalent to 0.1 to 1.0 U/ml), then 1ml of this diluted solution and 4ml of 0.1% soluble starch solution were put in a test tube to react appropriately at 37°C for 10 min, and immediately 5ml of Somogyi copper reagent was added, whereby production amount of glucose was determined by Somogyi's method and activity was finally obtained establishing $1\mu$ mol/min as 1 U.

Digestion test of soluble starch:

1ml of enzyme solution was added to 4ml of 10mg% soluble starch solution to react at 37°C for 3 hours, and only when decomposition percentage is 98% and over, the solution was qualified as the enzyme solution for this method.

Preparation of test solution:

If a sample includes oligosaccharides alone, they are extracted using water or ethanol, and if including polysaccharides, then oligosaccharides should be completely extracted by using 80% ethanol. If extraction has been carried out using 80% ethanol, then solvent should be eliminated in vacuo at 60°C, and residues be dissolved and diluted with water. An aqueous solution of 5 to 15 mg% as glocose is used as a solution to be tested (note that in case of using hydrogenated maltose sirup as sweetener, concentration of saccharide should be prepared to be 50 to 100 mg%).

Procedure:

Put 4ml of test solution in a test tube, 1ml of enzyme solution (3U) was added to the test solution to react at 37°C for 3 hours, then glucose was determined by adding 5ml of Somogi copper reagent according to Somogi method, whereby amount of glucose produced by glucoamylase was calculated.

(4) Measurement of Insoluble Starch

Preparation of test solution:

Oligosaccharides were completely extracted from a sample corresponding to 2.5 to 3.0g in dry solid basis using 80% ethanol, and residue was dispersed into 200ml of water (there is no need of measurement, if reaction with iodine performed by heating this solution is negative) so as to obtain hydrochloric acid of 2.5% concentration, then was heated in boiling water bath for 2.5 hours. After cooling, neutralizing and filtering, amount of the filtered solution was filled up to 250ml by water, and through further dilution when necessary, an aqueous solution of 15 to 15mg% as glucose was obtained as a solution to be tested.

Operation:

Adding 5ml of Somogi copper reagent to 5ml of test solution, determination was carried out as glucose by Somogyi method.

(5) Measurement of Sorbitol and Xylose Contents:

This measurement was carried out by means of gas chromatography as follows:
(A) Sample were methylated otherwise subject to trimethylsilylation.
(B) Any one of adipic acid-2-ethylhexyl ether, docosanoic acid, inositol or others was used as internal standard material.
(C) Operating conditions
   (a) Hydrogen flame ionization detector was used as a detector.
   (b) 2% QF-1 and Chromosorb W(AW-DMCS) or 3% SE-30 and Choromosorb W(AW-DMCS) was used as packing.
   (c) A stainless steel or glass column of 2m in length was used.
   (d) Calculation was effected in the aspect of peak area ratio.

Reference Example 1

2500kg of potato starch was put into a Ribbon Mixer, 250 liters of 1% hydrochloric acid was sprayed with compressed air while rotating the mixer, and after being uniformized through a mixer, further allowed to mature in the Ribbon-mixer for 10 hours. The obtained mixture was preliminarily dried up to a condition containing 3% of water, subsequently put into a Rotary-Kiln-Type converter to be continuously heated at a temperture of 180°C for two hours. It was acknowledged that the pyrodextrin thus obtained contained 58% dietary fibers.

4000 liters of water was added to this pyrodextrin to be a solution with its pH adjusted to 6.0 by adding 20% sodium hydroxide, then 0.2% by weight (by dry solid of the solution) of alpha-amylase (Thermamyl 60L produced by Novo Ind.) was added to hydrolyze at a temperature of 95°C for 1 hour. Most of the solution was then refined through normal process such as decoloring and filtration with activated charcoal, deionization with ion exchanger resins, and subsequently spray-dried. Thus, about 1800kg of maltodextrin having a caloric value of 168kcal/100g was obtained.

Reference Example 2

About 100 liters of residual solution hydrolyzed with alpha-amylase in the foregoing Reference Example 1 was heated at a temperature of 55°C, with its pH adjusted to 5.5, then was saccharified by adding 0.1% by weight of glucoamylase (produced by Daiwa Kasei Co.). The pH was then adjusted to 3.5 and reaction of glucoamylase was stopped. After refining in the same manner as Reference Example 1, 60kg of 50% solution was obtained through concentration. 100 liters of this solution was applied, at SV = 0.25, to a column filed with "XFS-43279.00" (produced by Dow Chemical Japan) which was an alkali metal type strongly acidic cation exchanger resin, then high molecular maltodextrin was extracted by applying water, and, after concentration, dextrin having a calorie value of 37kcal/100g was obtained by spray drying.

Reference Example 3

Adextrin solution was obtained using 2500kg corn starch sold on the market and treated in the same manner as Reference Example 1. The dextrin solution was further treated in the same manner as Reference Example 2, whereby high molecular maltodextrin whose caloric value was 39kca/100g per dry solid was obtained.

Followings are examples of low caloric foods each produced by normal method on the material mixture conditions shown below.

Example 1

Butter cookie:

|  | Low Caloric Food | Control |
|---|---|---|
| Wheat flour | 100    g | 100    g |
| Sugar | -- | 40    g |
| Dextrin obtained in Ref. Ex. 2 | 40    g | -- |
| Aspartame | 0.22 g | -- |
| Shortening | 45    g | 45    g |
| Water | 25    ml | 25    ml |
| Caloric value | 452kcal/100g | 532kcal/100g |

Example 2

American doughnut:

|  | Low calorie food | Control |
|---|---|---|
| Semi-hard wheat flour | 1350    g | 1350    g |
| Sugar | -- | 394    g |
| Dextrin obtained in Ref. Ex.3 | 394    g | -- |
| Aspartame | 2.2  g | -- |
| Whole egg | 300    g | 300    g |
| Cow's milk | 540    g | 540    g |
| Butter | 68    g | 68    g |
| Salt | a little | a little |
| Lemon flauor | proper quantity | proper quantity |
| Caloric value | 274kcal/100g | 332kcal/100g |

Example 3

Soft doughnut:

|  | Low calorie food | Control |
|---|---|---|
| Wheat flour | 100 g | 100 g |
| Dextrin obtained in Ref. Ex.2 | 35 g | -- |
| Aspartame | 0.19 g | -- |
| whole egg | 12 g | 12 g |
| Water | 45 g | 45 g |
| Skim soybean milk | 5 g | 5 g |
| Skim milk powder | 4 g | 4 g |
| Salt | 2 g | 2 g |
| Shortening | 6 g | 6 g |
| Baking powder | 4 g | 4 g |
| Caloric value | 188kcal/100g | 235kcal/100g |

Example 4

Pound cake:

|                           | Low Caloric Food | Control       |
| ------------------------- | ---------------- | ------------- |
| Wheat flour               | 100      g       |               |
| Sugar                     | --               | 125      g    |
| Dextrin obtained in Ref. Ex. 3 | 125      g  | --            |
| Aspartame                 | 0.69 g           | --            |
| Wole egg                  | 75       g       | 75       g    |
| Cow's milk                | 30       g       | 30       g    |
| Butter                    | 75       g       | 75       g    |
| Water                     | 25       g       | 25       g    |
| Caloric value             | 319kcal/100g     | 431kcal/100g  |

Example 5

Sponge cake:

|                           | Low Caloric Food | Control       |
| ------------------------- | ---------------- | ------------- |
| Wheat flour               | 100      g       | 100      g    |
| Whole egg                 | 100      g       | 100      g    |
| Sugar                     | --               | 100      g    |
| Dextrin obtained in Ref. Ex. 2 | 100      g  | --            |
| Aspartame                 | 0.55 g           | --            |
| Caloric value             | 190kcal/100g     | 309kcal/100g  |

Example 6

Custard cream:

| | Low Caloric Food | | Control | |
|---|---|---|---|---|
| Wheat flour | 14 | g | 14 | g |
| Corn starch | 14 | g | 14 | g |
| Cow's milk | 360 | g | 360 | g |
| Egg yolk | 70 | g | 70 | g |
| Butter | 4 | g | 4 | g |
| Sugar | -- | | 120 | g |
| Dextrin obtained in Ref. Ex. 1 | 120 | g | -- | |
| Aspartame | 0.66 g | | -- | |
| Caloric value | 137kcal/100g | | 183kcal/100g | |

11

Example 7

Butter cream:

|  | Low Caloric Food | Control |
|---|---|---|
| Butter | 50 g | 50 g |
| Shortening | 50 g | 50 g |
| Sugar | -- | 90 g |
| Dextrin obtained in Ref. Ex. 2 | 90 g | -- |
| Aspartame | 0.5 g | -- |
| Caloric value | 368kcal/100g | 623kcal/100g |

Example 8

Sweet chocolate:

|  | Low Caloric Food | Control |
|---|---|---|
| Bitter chocolate | 30 g | 30 g |
| Sugar | -- | 50 g |
| Dextrin obtained in Ref. Ex. 3 | 50 g | -- |
| Aspartame | 0.28 g | -- |
| Cacao butter | 10 g | 10 g |
| Caloric value | 366kcal/100g | 461kcal/100g |

Example 9

milk chocolate:

|  | Low Caloric Food | Control |
|---|---|---|
| Bitter chocolate | 30 g | 30 g |
| Sugar | -- | 35 g |
| Dextrin obtained in Ref. Ex. 2 | 35 g | -- |
| Aspartame | 0.2 g | -- |
| Milk powder | 28 g | 28 g |
| Caloric value | 372kcal/100g | 509kcal/100g |

Example 10

Pudding:

|  | Low Caloric Food | Control |
|---|---|---|
| Sugar | -- | 40 g |
| Dextrin obtained in Ref. Ex. 3 | 40 g | -- |
| Aspartame | 0.22 g | -- |
| Cow's milk | 480 g | 480 g |
| Whole egg | 200 g | 200 g |
| Water | 20 g | 20 g |
| Caloric value | 84kcal/100g | 103kcal/100g |

## Example 11

Bavarian:

|  | Low Caloric Food | | Control | |
|---|---|---|---|---|
| Cow's milk | 200 | g | 200 | g |
| Fresh cream | 200 | ml | 200 | ml |
| Egg york | 2 | pieces | 2 | pieces |
| Sugar | -- | | 60 | g |
| Dextrin obtained in Ref. Ex. 2 | 60 | g | -- | |
| Aspartame | 0.33 | g | -- | |
| Gelatin | 5 | g | 5 | g |
| Calorie value | 122kcal/100g | | 167kcal/100g | |

Examlpe 12

Orange jelly:

|  | Low Caloric Food | | Control | |
| --- | --- | --- | --- | --- |
| Sugar | -- | | 300 | g |
| Dextrin obtained in Ref. Ex. 3 | 300 | g | -- | |
| Aspartame | 1.65 | g | -- | |
| Water | 500 | g | 500 | g |
| Gelatin | 40 | g | 40 | g |
| Orange juice | 450 | ml | 450 | ml |
| Curacao | 150 | ml | 150 | ml |
| Caloric value | 70kcal/100g | | 122kcal/100g | |

Example 13

Hard yoghurt:

|  | Low Caloric Food | | Control | |
| --- | --- | --- | --- | --- |
| Skim milk | 1000 | g | 1000 | g |
| Sugar | -- | | 100 | g |
| Dextrin obtained in Ref. Ex. 3 | 100 | g | -- | |
| Stevioside | 0.5 | g | -- | |
| Flauor | small quantity | | small quantity | |
| Hardner | small quantity | | small quantity | |
| Caloric value | 58kcal/100g | | 88kcal/100g | |

Example 14

Ice cream:

|  | Low Caloric Food | Control |
|---|---|---|
| Butter | 6.5    g | 6.5    g |
| Unskimmed milk powder | 8      g | 8      g |
| Skim milk powder | 6.4    g | 6.4    g |
| Sugar | -- | 13      g |
| Dextrin obtained in Ref. Ex. 1 | 13      g | -- |
| Lycostevia S | 0.07 g | -- |
| Emulsifyier | 0.3    g | 0.3    g |
| Stabilizer | 0.2    g | 0.2    g |
| Water | 65.55 g | 65.55 g |
| Caloric value | 126kcal/100g | 155kcal/100g |

## Example 15

Orange conc. juice (1/5):

|  | Low Caloric Food | Control |
|---|---|---|
| Sugar | -- | 680 g |
| Dextrin obtained in Ref. Ex. 3 | 680 g | -- |
| Stevioside | 37.4 g | -- |
| Citric acid | 6 g | 6 g |
| Malic acid | 3 g | 3 g |
| Cloudy-base | 5 g | 5 g |
| Colorant | small quantity | small quantity |
| Unnshu 1/5 concentrated juice | 26 g | 26 g |
| Flavor | 5 ml | 5 ml |
| Water | 525 ml | 525 ml |
| Caloric value (after diluted) | 5kcal/100g | 44kcal/100g |

## Example 16

American milk shake:

| | Low Caloric Food | | Control | |
|---|---|---|---|---|
| Cow's milk | 20 | ml | 20 | ml |
| Butter | 5.9 | g | 5.9 | g |
| Skim milk powder | 13 | g | 13 | g |
| Sugar | -- | | 13 | g |
| Corn sirup solid powder | | | 15 | g |
| Dextrin obtained in Ref. Ex. 3 | 18.2 | g | -- | |
| Stevioside | 1 | g | | |
| Stabilizer | 0.3 | g | 0.3 | g |
| Emulsifyier | 0.3 | g | 0.3 | g |
| Water | 43.1 | ml | 43.1 | ml |
| Cow's milk (cold) | 200 | ml | 200 | ml |
| Caloric value | 77kcal/100g | | 98kcal/100g | |

Note: The above American milk shake was prepared by shaking, preliminarily preparing ice cream and cow's milk.

## Claims

1. A low calaric food or drink characterized by comprising a low caloric maltodextrin which is obtained by dissolving a pyrodextrin into water and reacting alpha-amylase on the pyrodextrin, said dextrin being prepared by heating a starch to which mineral acid is added.

2. A low caloric food or drink as set forth in claim 1, wherein said starch is a potato starch.

3. A low caloric food or drink as set forth in claim 1, wherein said low caloric maltodextrin is prepared through the steps of additionally reacting glucoamylase after reacting alpha-amylase on the dextrin; filtration and refining according to normal method; and removing a high caloric maltodextrin section alone by extracting it through either ion exchanger chromatography method or organic solvent method.

18

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 368 451 (MATSUTANI CHEMICAL INDUSTRIES CO. LTD.) <br> * page 2, line 20 - page 4, line 37 * | 1-3 | A23L1/09 <br> A23L1/308 |
| | ----- | | |
| | | | **TECHNICAL FIELDS SEARCHED (Int. Cl.5 )** |
| | | | A23L |

The present search report has been drawn up for all claims

| Place of search | Date of completion of the search | Examiner |
|---|---|---|
| BERLIN | 19 FEBRUARY 1992 | ALVAREZ ALVAREZ C. |

EPO FORM 1503 03.82 (P0401)